# EUROPEAN PATENT SPECIFICATION

(11) **EP 3 060 101 B1**
(45) Date of publication and mention of the grant of the patent: **23.05.2018**
(21) Application number: 14798994.1
(22) Date of filing: 16.10.2014
(51) Int. Cl.: A61B 5/00

(54) **SENSOR APPARATUS AND METHOD FOR MONITORING A VITAL SIGN OF A SUBJECT**
SENSORVORRICHTUNG UND VERFAHREN ZUR ÜBERWACHUNG DER VITALZEICHEN EINER PERSON
APPAREIL DE DÉTECTION ET PROCÉDÉ DE SURVEILLANCE D'UN SIGNE VITAL D'UN SUJET

(30) Priority: 22.10.2013 US 201361894025 P
(43) Date of publication of application: 31.08.2016
(73) Proprietor: Koninklijke Philips N.V., 5656 AE Eindhoven (NL)
(72) Inventor: KURZENBERGER, Heinz Otto, NL-5656 AE Eindhoven (NL); EMMRICH, Thomas Gerhard, NL-5656 AE Eindhoven (NL); WILM, Bernd Günter Werner, NL-5656 AE Eindhoven (NL); ZIMMERMANN, Steffen, NL-5656 AE Eindhoven (NL); GREINER, Harald, NL-5656 AE Eindhoven (NL)
(74) Representative: Ledeboer, Johannes Albertus
(86) International application number: PCT/IB2014/065348
(87) International publication number: WO 2015/059606

(56) References cited:
- WO-A1-2012/170305
- WO-A1-2013/028784
- WO-A2-2013/003787
- US-A- 4 796 634
- US-A- 6 049 727
- US-A1- 2009 069 642
- US-A1- 2010 063 438

## Description

### FIELD OF THE INVENTION

The present invention relates to a sensor apparatus for monitoring a vital sign of a subject. Further, the present invention relates to a patient monitoring apparatus and a patient monitoring system for monitoring vital signs of a subject. Still further, the present invention relates to corresponding methods and a computer program for implementing the method for monitoring vital signs of a subject.

### BACKGROUND OF THE INVENTION

Sensor measurements for measuring / monitoring a vital sign (e.g. heart rate, breathing (respiration) rate, Sp02, blood pressure, etc.) are conventionally either taken continuously (e.g. ECG) or aperiodic (e.g. NBP (non-invasive blood pressure)). Aperiodic measurements can be taken on demand by the user (e.g. by selecting a Start operation) or automatically, either in fixed intervals (e.g. AUTO = 30 min) or in a given (i.e. preprogrammed) sequence (e.g. 4x 15min, 2x 30min, then very 60 min). The sequence allows for a more flexible scheme. It may be selected after a rather simple surgery to check vital signs first more frequent and then extend the intervals progressively as the patient is expected to recover and needs less monitoring.

Allowing a more flexible sensing sequence instead of just measuring sensor signals at fixed intervals is an improvement for most cases. However, it is known that some patients tend to e.g. slow down with their respiration rate after anesthesia and then stop breathing completely. This can be detected with a full apnea monitoring, i.e. a continuous monitoring of respiration. However, such a measurement needs to be very sensitive and therefore is prone to noise and movement artifacts so that usually a lot of false alarms are generated. In addition such measurements need to run continuously and therefore require more energy than an aperiodic measurement even if it is running more frequently. US 4,796,634 discloses an apparatus according to the preamble of claim 1. US 6,049,727 discloses an in vivo implantable sensor that obtains spectra of body fluid constituents and processes the spectra to determine the concentration of a constituent of the body fluid. The sensor is fully implanted and is set in place to allow plural measurements to be taken at different time periods from a single in vivo position. A processor may signal to the implantable sensor via telemetry to make more frequent measurements if either the trending or the measured value of the constituents are determined to be problematic. The processor also detects "outlier data," which is data that falls outside of preset bounds due to a sensor malfunction. If outlier data occurs, the processor telemeters the sensor to take another measurement, and triggers an alarm if subsequent measurements are also outside of preset bounds.

### SUMMARY OF THE INVENTION

It is an object of the present invention to provide a sensor apparatus, patient monitoring apparatus and system as well as corresponding methods for monitoring a vital sign of a subject that are optimized with respect to energy consumption and performance of measurements.

In a first aspect of the present invention a sensor apparatus for monitoring a vital sign of a subject is presented as defined in claim 1.

In a further aspect of the present invention a patient monitoring apparatus is presented as defined in claim 12.

In yet further aspects of the present invention, there are provided corresponding methods, as well as a non-transitory computer-readable recording according to claim 15. Preferred embodiments of the invention are defined in the dependent claims. It shall be understood that the claimed apparatus, system, methods, computer program and medium have similar and/or identical preferred embodiments as the claimed sensor apparatus and as defined in the dependent claims.

The present invention is based on the idea to make the sensor apparatus more "intelligent" and to make the measurement intervals more flexible compared to the preprogrammed measurement intervals as conventionally use. Seamless and unobtrusive measurements can thus be used in more cases where otherwise conventional continuous intensive care measurements would be indicated. The proposed (aperiodic) sensor measurements are usually non-invasive, require less energy and therefore can be done wireless with smaller batteries. They can be designed to be usually less sensitive to noise and movement artifacts. All those factors contribute to ease of use and patient comfort. The present invention thus helps to further narrow the gap that exists in monitoring performance between those measurements and the conventional continuous high end measurements still required for critically ill patients in intensive care settings.

### BRIEF DESCRIPTION OF THE DRAWINGS

These and other aspects of the invention will be apparent from and elucidated with reference to the embodiment(s) described hereinafter. In the following drawings
Fig. 1 shows an embodiment of a sensor apparatus according to the present invention,
Fig. 2 shows an embodiment of a patient monitoring system and a patient monitoring apparatus according to the present invention,
Fig. 3 shows a flow chart of a sensing method according to the present invention, and
Fig. 4 shows a flow chart of a monitoring method according to the present invention.

### DETAILED DESCRIPTION OF THE INVENTION

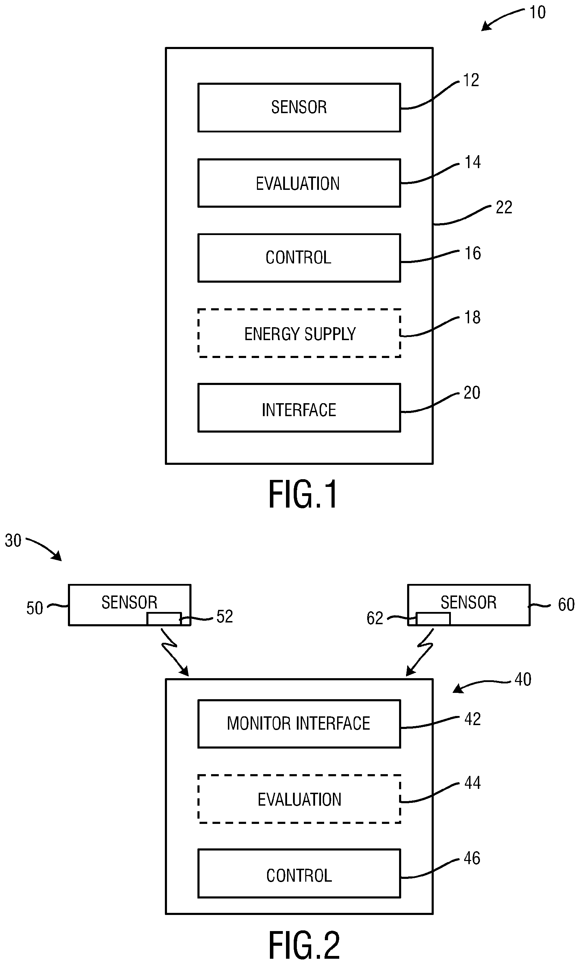

Fig. 1 shows an embodiment of a sensor apparatus 10 according to the present invention. It includes a sensor 12 that measures a sensor signal representing a vital sign or being related to a vital sign of a subject (e.g. a patient in a hospital, an elderly person at home or in a retirement home). Said sensor 12 may e.g. be respiration sensor for sensing the respiration rate, a blood pressure sensor for sensing the blood pressure, ECG electrodes for sensing an ECG, or a plethysmographic sensor; other examples of sensors exist. The sensor 12 is usually arranged at the patient's body, but may also be a sensor for contactless unobtrusive measurement. In other embodiments of the sensor apparatus two or more (identical or different) sensors are provided.

Further, an evaluation unit 14 is provided that evaluates the measured sensor signal to determine, based on previously measured sensor signals, changes of the health condition of the subject and/or to identify an unsuccessful measurement or an unreliable sensor signal. Said evaluation unit 14 is preferably implemented by an accordingly programmed processor or a dedicated hardware circuit. It may store previously measured sensor signals by itself, or receive those previously measured sensor signals from a memory that is internal or external to the sensor apparatus 10.

A control unit 16 is provided that controls said sensor 12 to automatically change the measurement interval based the result of evaluation performed by the evaluation unit 14. The control unit 16 is preferably implemented by a software or hardware controller or an accordingly programmed processor, e.g. the same processor that is used for the evaluation unit 14.

In some embodiments the sensor apparatus 10 is coupled to a central patient monitor (or another central unit) by a wired connection for data transfer (in particular of measured sensor signals to the central patient monitor) and for energy supply to the sensor apparatus. In other embodiments, however, the sensor apparatus 10 is coupled to a central patient monitor (or another central unit) by a wireless connection (e.g. using a WLAN, Zigbee or Bluetooth connection) for data transfer and includes its own energy supply unit 18, e.g. a rechargeable or non-rechargeable battery as indicated in Fig. 1 by dashed lines. For the (wired or wireless) connection an appropriated interface 20 is preferably provided.

The elements of the sensor apparatus 10 are preferably housed within a common housing 22, but may generally also be arranged in distributed manner and housed in different housings.

Preferably, in one advantageous implementation a method for aperiodic measurements is enabled to enter automatically a more frequent measurement mode dependent on the previously measured sensor value(s), i.e. if they indicate a worsening condition of the patient. In such a "frequent" measurement mode additional measurements are automatically taken in between the preprogrammed intervals. The extra number of measurements and/or the time between measurements can be automatically adjusted, for instance according of the prediction of the severity of the deterioration (e.g. prediction on when an alarm limit will be reached).

Thus, in an embodiment the control unit 16 is configured to control said sensor 12 to increase the measurement interval if the health condition of the subject is improving and/or to decrease the measurement interval if the health condition of the subject is worsening. Thus, the health condition of the subject directly has an influence on the automatic adjustment of the measurement interval.

In the first case (i.e. if the health status is increasing) or if the health status is stable (i.e. if the subject's health condition is neither improving nor worsening) it is preferred, not to increase the measurement interval above an upper limit set e.g. in a predetermined measurement scheme or prescribed by security rules. Thus, in case of an improvement of the subject's health condition the measurement interval is often maintained as it is. Hence, the control unit 16 is preferably configured to control the sensor 12 to measure a sensor signal at least at a minimum measurement interval.

The control unit 16 may also be configured to control the degree by which the measurement interval is changed based on the severity of the change of the health condition of the subject. Thus, if for instance the health condition is strongly decreasing the measurement interval will be reduced more strongly compared to a situation in which the health condition is only slightly decreasing, in which the measurement interval will be reduced only moderately.

Preferably, the control unit 16 is configured to control the sensor 12 to issue one of the measured sensor signals (i.e. measured within a predetermined measurement interval, e.g. within 5 min) as a kind of preliminary sensor signal, even if all those measurements were unsuccessful or all those measured sensor signals are unreliable. Measurement of the sensor signal is then repeated for an additional predetermined period (e.g. 10 min), which additional measurements may be taken "in the background" so that the user does not necessarily notice of it. The issued (preliminary) sensor signal may finally be replaced by a new sensor signal measured during said additional predetermined period, if during said additional predetermined period measurement was successful or a reliable sensor signal has been measured. Otherwise, the preliminary sensor signal is not replaced.

In another embodiment the measurement of the sensor signal may be started earlier, i.e. in advance of the planned measurement time (e.g. in a pre-measurement window) to have a sensor signal available when the measurement is due according to the planned measurement time and measurement interval.

For determining if the health condition of the subject has changed various options exists for the evaluation unit 14. In one embodiment it is determined if the measured sensor signal deviates by at least a first predetermined absolute or relative amount from one or more last measured sensor signals. In another embodiment it is determined if the measured sensor signal deviates by at least a first predetermined absolute or relative amount from an expected or estimated sensor signal trend line determined from the two or more last measured sensor signals. In still another embodiment a comparison is made of the measured sensor signal with one or more sensor signals measured by other sensors. Still further, in an embodiment information received from an external source, such as other health status sources (e.g. an early warning score from a patient monitoring apparatus) is used to determine, based on the measured sensor signal, if the health condition of the subject is improving or worsening.

In still further embodiments the evaluation unit 14 is configured to determine that the health condition of the subject has changed if a health status signal derived from said measured sensor signal deviates by at least a first predetermined absolute or relative amount from the current health status signal, from an expected or estimated health status trend line determined from the two or more last health status signals and/or from information received from another source.

In another advantageous implementation a method to retry automatically a measurement is enabled, if that measurement did not succeed, i.e. a reliable measurement could not be obtained at the automatically defined time or within a predefined time period. Hence, in such an implementation the control unit 16 is configured to control the sensor 12 to repeat measurement of the sensor signal for a predetermined period, representing a predetermined time duration or a predetermined number of times, or until the measurement is successful or the sensor signal is reliable, if an unsuccessful measurement or an unreliable sensor signal has been identified.

To determine that a measurement has been unsuccessful or that a sensor signal is unreliable said evaluation unit is preferably configured to determine if no sensor signal could be measured or if the measured sensor signal deviates by at least a second predetermined absolute or relative amount from one or more last measured sensor signals, from an expected or estimated sensor signal trend line determined from the two or more last measured sensor signals or from at least one sensor signal measured by one or more other sensors.

The first or second predetermined absolute or relative amounts and the various trend lines used in the above mentioned embodiments may be obtained empirically from earlier measurements or set by the user.

Fig. 2 shows an embodiment of a patient monitoring system 30 and a patient monitoring apparatus 40 for monitoring vital signs of a subject according to the present invention. The patient monitoring system 30 comprises said (central) patient monitoring apparatus 40 and one or more sensors 50, 60 for measuring / monitoring one or more vital signs of a subject.

The patient monitoring apparatus 40 comprises a monitor interface 42 that receives sensor signals representing a vital sign or being related to a vital sign measured by said sensors 50, 60 and transmits control information to said sensors 50, 60. An evaluation unit 44 is provided to evaluate received sensor signals to determine, based on previously received sensor signals, changes of the health condition of the subject and/or to identify an unsuccessful measurement or an unreliable sensor signal. Said evaluation unit 44 thus generally performs the same task as the evaluation unit 14 of the sensor apparatus, but performs this task for one or more sensors as a common evaluation unit. Further, a control unit 46 is provided to generate control information for control of said sensors 50, 60 to automatically change the measurement interval based the result of said evaluation. Said control unit 46 thus generally performs the same task as the control unit 16 of the sensor apparatus, but performs this task for one or more sensors as a common control unit.

While the sensors 50, 60 may generally be part of a sensor apparatus as explained above, but generally only the sensors themselves (i.e. without associated evaluation unit and control unit) are provided in said patient monitoring system 30. Said sensors 50, 60 are thus configured to measure a sensor signal representing a vital sign or being related to a vital sign. The sensors 50, 60 comprise a sensor interface 52, 62 for transmitting measured sensor signals to said patient monitoring apparatus 40 and for receiving control information from said patient monitoring apparatus 40.

It shall be noted that the patient monitoring apparatus and its elements can be further configured and has similar preferred embodiments as the sensor apparatus and as explained above.

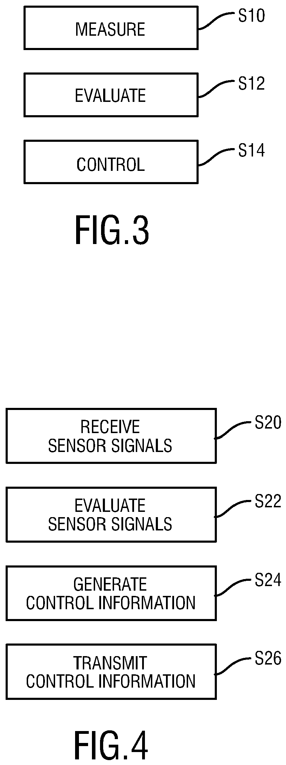

Fig. 3 shows a flow chart of a sensing method for monitoring a vital sign of a subject according to the present invention, as performed by a sensor apparatus 10 shown in Fig. 1. The first step S10 includes measuring a sensor signal representing a vital sign or being related to a vital sign. The second step S12 includes evaluating said sensor signal to determine, based on previously measured sensor signals, changes of the health condition of the subject and/or to identify an unsuccessful measurement or an unreliable sensor signal. The third step S14 includes controlling said measurement of the sensor signal to automatically change the measurement interval based the result of said evaluation.

Fig. 4 shows a flow chart of a monitoring method for monitoring vital signs of a subject according to the present invention, as performed by a patient monitoring apparatus 40 shown in Fig. 2. The first step S20 includes receiving sensor signals representing a vital sign or being related to a vital sign measured by at least one sensor. The second step S22 includes evaluating received sensor signals to determine, based on previously received sensor signals, changes of the health condition of the subject and/or to identify an unsuccessful measurement or an unreliable sensor signal. The third step S24 includes generating control information for control of said at least one sensor to automatically change the measurement interval based the result of said evaluation. The fourth step S26 includes transmitting said control information to said at least one sensor.

In summary, the general concept of the present invention applies a dynamic measurement interval depending on the patient's health condition. The trigger to change measurement interval is preferably a worsening condition of the patient.

A worsening condition can be defined if a current or averaged value (e.g. respiration rate) is above a configurable limit, representing e.g. an alarm limit of the measurement. The limit can also be dynamically adjusted depending on other patient conditions like activity or posture (e.g. if the patient is climbing up stairs and it is expected that respiration rate goes up). Therefor the limit for switching the measurement interval is adapted to be more insensitive in such a case. The evaluation unit is thus preferably configured to dynamically adjust the first and/or second predetermined absolute or relative amount depending on a physical condition of the subject, in particular to dynamically adjust the first and/or second predetermined absolute or relative amount depending on whether or not the status is performing a physical activity and/or the kind of physical activity of the subject.

A worsening condition can also be defined as change of measured value over time (trend).

The measurement interval is made longer when the patient's health condition improves to increase the sensor operating time. The measurement interval is made shorter when the patient's health condition gets worse to detect critical events earlier compared to a static measurement regime having fixed measurement intervals.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Sensor apparatus for monitoring a vital sign of a subject, comprising:
at least one sensor (12, 50, 60) configured to measure a sensor signal representing a vital sign or being related to a vital sign,
an evaluation unit (14) configured to evaluate said sensor signal to determine, based on previously measured sensor signals, changes of the health condition of the subject and to identify an unsuccessful measurement or an unreliable sensor signal, and
a control unit (16) configured to control said at least one sensor to automatically change the measurement interval based on the result of said evaluation, **characterised in that** said control unit (16) is configured to control said at least one sensor
- to repeat measurement of the sensor signal for a predetermined period, representing a predetermined time duration or a predetermined number of times, or until the measurement is successful or the sensor signal is reliable, if an unsuccessful measurement or an unreliable sensor signal has been identified,
- to issue one of the measured sensor signals, even if all measurements were unsuccessful or all measured sensor signals are unreliable; and wherein,
if all measurements were unsuccessful or all measured sensor signals are unreliable, the control unit (16) is further configured to control said at least one sensor
- to repeat measurement of the sensor signal for an additional predetermined period, and
- to replace the issued sensor signal by a new sensor signal measured during said additional predetermined period, if during said additional predetermined period measurement was successful or a reliable sensor signal has been measured.

2. Sensor apparatus as claimed in claim 1,
wherein said control unit (16) is configured to control said at least one sensor to increase the measurement interval if the health condition of the subject is improving and/or to decrease the measurement interval if the health condition of the subject is worsening.

3. Sensor apparatus as claimed in claim 2,
wherein said control unit (16) is configured to control said at least one sensor to measure the sensor signal at least at a minimum measurement interval or to control the degree by which the measurement interval is changed based on the severity of the change of the health condition of the subject.

4. Sensor apparatus as claimed in claim 1, further comprising
an energy supply unit (18), in particular a battery or accumulator, for energy supply of said sensor apparatus, and
a housing (22) that houses said at least one sensor, said evaluation unit, said control unit and said energy supply unit.

5. Sensor apparatus as claimed in claim 1,
wherein said evaluation unit (14) is configured to determine that the health condition of the subject has changed if the measured sensor signal deviates by at least a first predetermined absolute or relative amount from one or more last measured sensor signals, from an expected or estimated sensor signal trend line determined from the two or more last measured sensor signals, from one or more sensor signal measured by other sensors and/or from information received from an external source.

6. Sensor apparatus as claimed in claim 1,
wherein said evaluation unit (14) is configured to determine that the health condition of the subject has changed if a health status signal derived from said measured sensor signal deviates by at least a first predetermined absolute or relative amount from the current health status signal, from an expected or estimated health status trend line determined from the two or more last health status signals and/or from information received from another source.

7. Sensor apparatus as claimed in claim 1,
wherein said evaluation unit (14) is configured to determine that a measurement has been unsuccessful or that a sensor signal is unreliable if no sensor signal could be measured or if the measured sensor signal deviates by at least a second predetermined absolute or relative amount from one or more last measured sensor signals, from an expected or estimated sensor signal trend line determined from the two or more last measured sensor signals or from at least one sensor signal measured by one or more other sensors.

8. Sensor apparatus as claimed in claim 5,
wherein said evaluation unit (14) is configured to dynamically adjust the first and/or second predetermined absolute or relative amount depending on a physical condition of the subject.

9. Sensor apparatus as claimed in claim 8,
wherein said evaluation unit (14) is configured to dynamically adjust the first and/or second predetermined absolute or relative amount depending on whether or not the status is performing a physical activity and/or the kind of physical activity of the subject.

10. Sensor apparatus as claimed in claim 2,
wherein said control unit (16) is configured to control said at least one sensor to start a measurement in advance of a planned measurement time.

11. Method of monitoring a vital sign of a subject, comprising:
measuring a sensor signal representing a vital sign or being related to a vital sign,
evaluating said sensor signal to determine, based on previously measured sensor signals, an unsuccessful measurement or an unreliable sensor signal, and
controlling said measurement of the sensor signal to automatically change the measurement interval based on the result of said evaluation, **characterised in that** said controlling is configured to control said measurement
- to repeat measurement of the sensor signal for a predetermined period, representing a predetermined time duration or a predetermined number of times, or until the measurement is successful or the sensor signal is reliable, if an unsuccessful measurement or an unreliable sensor signal has been identified,
- to issue one of the measured sensor signals, even if all measurements were unsuccessful or all measured sensor signals are unreliable; and wherein, if all measurements were unsuccessful or all measured sensor signals are unreliable, the controlling is further configured to control said measurement
- to repeat measurement of the sensor signal for an additional predetermined period, and
- to replace the issued sensor signal by a new sensor signal measured during said additional predetermined period, if during said additional predetermined period measurement was successful or a reliable sensor signal has been measured.

12. Patient monitoring apparatus for monitoring vital signs of a subject, comprising
a monitor interface (42) configured to receive sensor signals representing a vital sign or being related to a vital sign measured by at least one sensor and for transmitting control information to said at least one sensor,
an evaluation unit (44) configured to evaluate received sensor signals to determine, based on previously received sensor signals, changes of the health condition of the subject and to identify an unsuccessful measurement or an unreliable sensor signal, and
a control unit (46) configured to generate control information for control of said at least one sensor to automatically change the measurement interval based on the result of said evaluation, **characterised in that** said control unit (46) is configured to generate control information to control said at least one sensor
- to repeat measurement of the sensor signal for a predetermined period, representing a predetermined time duration or a predetermined number of times, or until the measurement is successful or the sensor signal is reliable, if an unsuccessful measurement or an unreliable sensor signal has been identified,
- to issue one of the measured sensor signals, even if all measurements were unsuccessful or all measured sensor signals are unreliable; and wherein, if all measurements were unsuccessful or all measured sensor signals are unreliable, the control unit (16) is further configured to control said at least one sensor
- to repeat measurement of the sensor signal for an additional predetermined period, and
- to replace the issued sensor signal by a new sensor signal measured during said additional predetermined period, if during said additional predetermined period measurement was successful or a reliable sensor signal has been measured.

13. Method of monitoring vital signs of a subject, comprising
receiving sensor signals representing a vital sign or being related to a vital sign measured by at least one sensor,
evaluating received sensor signals to determine, based on previously received sensor signals, an unsuccessful measurement or an unreliable sensor signal,
generating control information for control of said at least one sensor to automatically change the measurement interval based the result of said evaluation, and
transmitting said control information to said at least one sensor,
wherein control information is generated to control said at least one sensor
- to repeat measurement of the sensor signal for a predetermined period, representing a predetermined time duration or a predetermined number of times, or until the measurement is successful or the sensor signal is reliable, if an unsuccessful measurement or an unreliable sensor signal has been identified,
- to issue one of the measured sensor signals, even if all measurements were unsuccessful or all measured sensor signals are unreliable,
- to repeat measurement of the sensor signal for an additional predetermined period, and
- to replace the issued sensor signal by a new sensor signal measured during said additional predetermined period, if during said additional predetermined period measurement was successful or a reliable sensor signal has been measured.

14. Patient monitoring system for monitoring vital signs of a subject, comprising
a patient monitoring apparatus (40) as claimed in claim 12, and
at least one sensor (10, 50, 60) configured to measure a sensor signal representing a vital sign or being related to a vital sign, said at least one sensor comprising a sensor interface for transmitting measured sensor signals to said patient monitoring apparatus and for receiving control information from said patient monitoring apparatus.

15. Computer readable non-transitory medium having instructions stored thereon which, when carried out on a computer, cause the computer to perform the steps of the method as claimed in claim 13.

## Patentansprüche

1. Sensorvorrichtung zur Überwachung eines Vitalzeichens eines Patienten, umfassend:
wenigstens einen Sensor (12, 50, 60), der so konfiguriert ist, dass er ein Sensorsignal misst, das ein Vitalzeichen darstellt oder mit einem Vitalzeichen zusammenhängt,
eine Auswerteeinheit (14), die so konfiguriert ist, dass sie das Sensorsignal auswertet, um basierend auf zuvor gemessenen Sensorsignalen Änderungen des Gesundheitszustands des Patienten zu bestimmen und eine erfolglose Messung oder ein unzuverlässiges Sensorsignal zu identifizieren, und
eine Steuereinheit (16), die konfiguriert ist, um den wenigstens einen Sensor so zu steuern, dass das Messintervall basierend auf dem Ergebnis der Auswertung geändert wird,
**dadurch gekennzeichnet,**
**dass** die Steuereinheit (16) so konfiguriert ist, dass sie den wenigstens einen Sensor steuert, um
- die Messung des Sensorsignals für einen vorbestimmten Zeitraum zu wiederholen, der eine vorbestimmte Zeitdauer oder eine vorbestimmte Anzahl von Malen darstellt, oder bis die Messung erfolgreich ist oder das Sensorsignal zuverlässig ist, wenn eine erfolglose Messung oder ein unzuverlässiges Sensorsignal identifiziert worden ist,
- eines der gemessenen Sensorsignale selbst dann auszugeben, wenn alle Messungen erfolglos waren oder alle gemessenen Sensorsignale unzuverlässig sind; und wobei,
wenn alle Messungen erfolglos waren oder alle gemessenen Sensorsignale unzuverlässig sind, die Steuereinheit (16) weiterhin so konfiguriert ist, dass sie den wenigstens einen Sensor steuert, um
- die Messung des Sensorsignals für einen zusätzlichen vorbestimmten Zeitraum zu wiederholen, und
- das ausgegebene Sensorsignal durch ein neues Sensorsignal zu ersetzen, das während des zusätzlichen vorbestimmten Zeitraums gemessen wurde, wenn während des zusätzlichen vorbestimmten Zeitraums die Messung erfolgreich war oder ein zuverlässiges Sensorsignal gemessen worden ist.

2. Sensorvorrichtung nach Anspruch 1,
wobei die Steuereinheit (16) so konfiguriert ist, dass sie den wenigstens einen Sensor so steuert, dass das Messintervall verlängert wird, wenn der Gesundheitszustand des Patienten sich verbessert, und/oder das Messintervall verkürzt wird, wenn der Gesundheitszustand des Patienten sich verschlechtert.

3. Sensorvorrichtung nach Anspruch 2,
wobei die Steuereinheit (16) so konfiguriert ist, dass sie den wenigstens einen Sensor so steuert, dass das Sensorsignal wenigstens bei einem Mindestmessintervall gemessen wird, oder den Grad steuert, um den das Messintervall basierend auf der Schwere der Änderung des Gesundheitszustands des Patienten geändert wird.

4. Sensorvorrichtung nach Anspruch 1, weiterhin umfassend
eine Energieversorgungseinheit (18), insbesondere eine Batterie oder einen Akkumulator, zur Energieversorgung der Sensorvorrichtung, und
ein Gehäuse (22), das den wenigstens einen Sensor, die Auswerteeinheit, die Steuereinheit und die Energieversorgungseinheit aufnimmt.

5. Sensorvorrichtung nach Anspruch 1,
wobei die Auswerteeinheit (14) so konfiguriert ist, dass sie bestimmt, dass der Gesundheitszustand des Patienten sich geändert hat, wenn das gemessene Sensorsignal um wenigstens einen vorbestimmten absoluten oder relativen Betrag von einem oder mehreren zuletzt gemessenen Sensorsignalen, von einer erwarteten oder geschätzten Sensorsignaltrendlinie, die aus den zwei oder mehr zuletzt gemessenen Sensorsignalen bestimmt worden ist, von einem oder mehreren Sensorsignalen, die von anderen Sensoren gemessen worden sind, und/oder von Informationen, die von einer externen Quelle empfangen worden sind, abweicht.

6. Sensorvorrichtung nach Anspruch 1,
wobei die Auswerteeinheit (14) so konfiguriert ist, dass sie bestimmt, dass der Gesundheitszustand des Patienten sich geändert hat, wenn ein vom gemessenen Sensorsignal stammendes Gesundheitsstatussignal um wenigstens einen ersten vorbestimmten absoluten oder relativen Betrag vom gegenwärtigen Gesundheitsstatussignal, von einer erwarteten oder geschätzten Gesundheitsstatustrendlinie, die aus den zwei oder mehr letzten Gesundheitsstatussignalen bestimmt worden ist, und/oder von Informationen, die von einer externen Quelle empfangen worden sind, abweicht.

7. Sensorvorrichtung nach Anspruch 1,
wobei die Auswerteeinheit (14) so konfiguriert ist, dass sie bestimmt, dass eine Messung erfolglos gewesen ist oder dass ein Sensorsignal unzuverlässig ist, wenn kein Sensorsignal gemessen werden konnte oder wenn das gemessene Sensorsignal um wenigstens einen zweiten vorbestimmten absoluten oder relativen Betrag von dem einen oder den mehreren zuletzt gemessenen Sensorsignalen, von einer erwarteten oder geschätzten Sensorsignaltrendlinie, die aus den zwei oder mehr zuletzt gemessenen Sensorsignalen bestimmt worden ist, oder von dem wenigstens einen Sensorsignal, das von dem einen oder den mehreren anderen Sensoren gemessen worden sind, abweicht.

8. Sensorvorrichtung nach Anspruch 5,
wobei die Auswerteeinheit (14) so konfiguriert ist, dass sie den ersten und/oder zweiten vorbestimmten absoluten oder relativen Betrag in Abhängigkeit von einem physischen Zustand des Patienten dynamisch einstellt.

9. Sensorvorrichtung nach Anspruch 8,
wobei die Auswerteeinheit (14) so konfiguriert ist, dass sie den ersten und/oder zweiten vorbestimmten absoluten oder relativen Betrag in Abhängigkeit davon, ob der Status eine physische Aktivität durchführt oder nicht, und/oder der Art der physischen Aktivität des Patienten dynamisch einstellt.

10. Sensorvorrichtung nach Anspruch 2,
wobei die Steuereinheit (16) so konfiguriert ist, dass wie den wenigstens einen Sensor steuert, um eine Messung vor einem geplanten Messzeitpunkt zu starten.

11. Verfahren zur Überwachung eines Vitalzeichens eines Patienten, umfassend:
das Messen eines Sensorsignals, das ein Vitalzeichen darstellt oder mit einem Vitalzeichen zusammenhängt,
das Auswerten des Sensorsignals, um basierend auf zuvor gemessenen Sensorsignalen eine erfolglose Messung oder ein unzuverlässiges Sensorsignal zu bestimmen, und
das Steuern der Messung des Sensorsignals, um das Messintervall basierend auf dem Ergebnis der Auswertung automatisch zu ändern,
**dadurch gekennzeichnet,dass** die Steuerung so konfiguriert ist, dass sie die Messung steuert, um
- die Messung des Sensorsignals für einen vorbestimmten Zeitraum zu wiederholen, der eine vorbestimmte Zeitdauer oder eine vorbestimmte Anzahl von Malen darstellt, oder bis die Messung erfolgreich ist oder das Sensorsignal zuverlässig ist, wenn eine erfolglose Messung oder ein unzuverlässiges Sensorsignal identifiziert worden ist,
- eines der gemessenen Sensorsignale selbst dann auszugeben, wenn alle Messungen erfolglos waren oder alle gemessenen Sensorsignale unzuverlässig sind; und wobei, wenn alle Messungen erfolglos waren oder alle gemessenen Sensorsignale unzuverlässig sind, die Steuerung weiterhin so konfiguriert ist, dass sie die Messung steuert, um
- die Messung des Sensorsignals für einen zusätzlichen vorbestimmten Zeitraum zu wiederholen, und
- das ausgegebene Sensorsignal durch ein neues Sensorsignal zu ersetzen, das während des zusätzlichen vorbestimmten Zeitraums gemessen wurde, wenn während des zusätzlichen vorbestimmten Zeitraums die Messung erfolgreich war oder ein zuverlässiges Sensorsignal gemessen worden ist.

12. Patientenüberwachungsvorrichtung zum Überwachen von Vitalzeichen eines Patienten, umfassend
eine Monitorschnittstelle (42), die so konfiguriert ist, dass sie Sensorsignale empfängt, die ein Vitalzeichen darstellen oder mit einem Vitalzeichen zusammenhängen, das von wenigstens einem Sensor gemessen wird, und Steuerungsinformationen zum wenigstens einen Sensor überträgt,
eine Auswerteeinheit (44), die so konfiguriert ist, dass sie empfangene Sensorsignale auswertet, um basierend auf zuvor empfangenen Sensorsignalen Änderungen des Gesundheitszustands des Patienten zu bestimmen und eine erfolglose Messung oder ein unzuverlässiges Sensorsignal zu identifizieren, und
eine Steuereinheit (46), die so konfiguriert ist, dass sie Steuerungsinformationen zur Steuerung des wenigstens einen Sensors erzeugt, um das Messintervall basierend auf dem Ergebnis der Auswertung zu ändern,
**dadurch gekennzeichnet,**
**dass** die Steuereinheit (46) konfiguriert ist, um Steuerungsinformationen zur Steuerung des wenigstens einen Sensors zu erzeugen, um
- die Messung des Sensorsignals für einen vorbestimmten Zeitraum zu wiederholen, der eine vorbestimmte Zeitdauer oder eine vorbestimmte Anzahl von Malen darstellt, oder bis die Messung erfolgreich ist oder das Sensorsignal zuverlässig ist, wenn eine erfolglose Messung oder ein unzuverlässiges Sensorsignal identifiziert worden ist,
- eines der gemessenen Sensorsignale selbst dann auszugeben, wenn alle Messungen erfolglos waren oder alle gemessenen Sensorsignale unzuverlässig sind; und wobei, wenn alle Messungen erfolglos waren oder alle gemessenen Sensorsignale unzuverlässig sind, die Steuereinheit (16) weiterhin so konfiguriert ist, dass sie den wenigstens einen Sensor steuert,
- die Messung des Sensorsignals für einen zusätzlichen vorbestimmten Zeitraum zu wiederholen, und
- das ausgegebene Sensorsignal durch ein neues Sensorsignal zu ersetzen, das während des zusätzlichen vorbestimmten Zeitraums gemessen wurde, wenn während des zusätzlichen vorbestimmten Zeitraums die Messung erfolgreich war oder ein zuverlässiges Sensorsignal gemessen worden ist.

13. Verfahren zur Überwachung von Vitalzeichen eines Patienten, umfassend
das Empfangen von Sensorsignalen, die ein Vitalzeichen darstellen oder mit einem Vitalzeichen zusammenhängen, das von wenigstens einem Sensor gemessen wird,
das Auswerten von empfangenen Sensorsignalen, um basierend auf zuvor gemessenen Sensorsignalen eine erfolglose Messung oder ein unzuverlässiges Sensorsignal zu bestimmen,
das Erzeugen von Steuerungsinformationen zur Steuerung des wenigstens einen Sensors, um das Messintervall basierend auf dem Ergebnis der Auswertung zu ändern, und
das Übertragen der Steuerungsinformationen zu dem wenigstens einen Sensor,
wobei die Steuerungsinformationen erzeugt werden, um den wenigstens einen Sensor zu steuern
- die Messung des Sensorsignals für einen vorbestimmten Zeitraum zu wiederholen, der eine vorbestimmte Zeitdauer oder eine vorbestimmte Anzahl von Malen darstellt, oder bis die Messung erfolgreich ist oder das Sensorsignal zuverlässig ist, wenn eine erfolglose Messung oder ein unzuverlässiges Sensorsignal identifiziert worden ist,
- eines der gemessenen Sensorsignale selbst dann auszugeben, wenn alle Messungen erfolglos waren oder alle gemessenen Sensorsignale unzuverlässig sind,
- die Messung des Sensorsignals für einen zusätzlichen vorbestimmten Zeitraum zu wiederholen, und
- das ausgegebene Sensorsignal durch ein neues Sensorsignal zu ersetzen, das während des zusätzlichen vorbestimmten Zeitraums gemessen wurde, wenn während des zusätzlichen vorbestimmten Zeitraums die Messung erfolgreich war oder ein zuverlässiges Sensorsignal gemessen worden ist.

14. Patientenüberwachungssystem zum Überwachen von Vitalzeichen eines Patienten, umfassend
eine Patientenüberwachungsvorrichtung (40) nach Anspruch 12 und
wenigstens einen Sensor (10, 50, 60), der konfiguriert ist, um ein Sensorsignal zu messen, das ein Vitalzeichen darstellt oder mit einem Vitalzeichen zusammenhängt, wobei der wenigstens eine Sensor eine Sensorschnittstelle zum Übertragen der gemessenen Sensorsignale zur Patientenüberwachungsvorrichtung und zum Empfang von Steuerungsinformationen von der Patientenüberwachungsvorrichtung umfasst.

15. Computerlesbares nichttransitorisches Medium mit darauf gespeicherten Anweisungen, die, wenn sie auf einem Computer ausgeführt werden, bewirken, dass der Computer die Schritte des Verfahrens nach Anspruch 13 durchführt.

## Revendications

1. Appareil de détection pour surveiller un signe vital d'un sujet, comprenant :
au moins un capteur (12, 50, 60) configuré pour mesurer un signal de capteur représentant un signe vital ou étant lié à un signe vital,
une unité d'évaluation (14) configurée pour évaluer ledit signal de capteur pour déterminer, sur la base de signaux de capteur préalablement mesurés, des changements de l'état de santé du sujet et pour identifier une mesure non concluante ou un signal de capteur non fiable, et
une unité de commande (16) configurée pour commander audit au moins un capteur de changer automatiquement l'intervalle de mesure sur la base du résultat de ladite évaluation,
**caractérisé en ce que**
ladite unité de commande (16) est configurée pour commander audit au moins un capteur
- de répéter la mesure du signal de capteur pendant une période prédéterminée, représentant une durée de temps prédéterminée ou un nombre de fois prédéterminé, ou jusqu'à ce que la mesure soit concluante ou que le signal de capteur soit fiable, si une mesure non concluante ou un signal de capteur non fiable a été identifié(e),
- d'émettre l'un des signaux de capteur mesurés, même si toutes les mesures ont été non concluantes ou si tous les signaux de capteur mesurés sont non fiables ; et dans lequel,
si toutes les mesures ont été non concluantes ou si tous les signaux de capteur mesurés sont non fiables, l'unité de commande (16) est configurée en outre pour commander audit au moins un capteur
- de répéter la mesure du signal de capteur pendant une période prédéterminée supplémentaire, et
- de remplacer le signal de capteur émis par un nouveau signal de capteur mesuré au cours de ladite période prédéterminée supplémentaire, si au cours de ladite période prédéterminée supplémentaire la mesure a été concluante ou si un signal de capteur fiable a été mesuré.

2. Appareil de détection selon la revendication 1,
dans lequel ladite unité de commande (16) est configurée pour commander audit au moins un capteur d'augmenter l'intervalle de mesure si l'état de santé du sujet s'améliore et/ou de réduire l'intervalle de mesure si l'état de santé du sujet empire.

3. Appareil de détection selon la revendication 2,
dans lequel ladite unité de commande (16) est configurée pour commander audit au moins un capteur de mesurer le signal de capteur au moins à un intervalle de mesure minimal ou de commander le degré de changement de l'intervalle de mesure sur la base de l'intensité du changement de l'état de santé du sujet.

4. Appareil de détection selon la revendication 1, comprenant en outre
une unité d'alimentation en énergie (18), en particulier une batterie ou un accumulateur, pour l'alimentation en énergie dudit appareil de détection, et
un logement (22) qui loge ledit au moins un capteur, ladite unité d'évaluation, ladite unité de commande et ladite unité d'alimentation en énergie.

5. Appareil de détection selon la revendication 1,
dans lequel ladite unité d'évaluation (14) est configurée pour déterminer que l'état de santé du sujet a changé si le signal de capteur mesuré s'écarte d'au moins une première valeur absolue ou relative par rapport à un ou plusieurs derniers signaux de capteur mesurés, par rapport à une ligne de tendance de signal de capteur attendu ou estimé déterminée à partir de deux derniers signaux de capteur mesurés ou plus, par rapport à un ou plusieurs signaux de capteur mesurés par d'autres capteurs et/ou par rapport à des informations reçues d'une source externe.

6. Appareil de détection selon la revendication 1,
dans lequel ladite unité d'évaluation (14) est configurée pour déterminer que l'état de santé du sujet a changé si un signal d'état de santé dérivé dudit signal de capteur mesuré s'écarte d'au moins une première valeur absolue ou relative prédéterminée par rapport au signal d'état de santé actuel, par rapport à une ligne de tendance d'état de santé attendu ou estimé déterminée à partir des deux derniers signaux d'état de santé ou plus, et/ou par rapport à des d'informations reçues d'une autre source.

7. Appareil de détection selon la revendication 1,
dans lequel ladite unité d'évaluation (14) est configurée pour déterminer qu'une mesure a été non concluante ou qu'un signal de capteur est non fiable si aucun signal de capteur n'a pu être mesuré ou si le signal de capteur mesuré s'écarte d'au moins une seconde valeur absolue ou relative prédéterminée par rapport à un ou plusieurs derniers signaux de capteur mesurés, par rapport à une ligne de tendance de signal de capteur attendu ou estimé déterminée à partir des deux derniers signaux de capteur mesurés ou plus ou par rapport à au moins un signal de capteur mesuré par un ou plusieurs autres capteurs.

8. Appareil de détection selon la revendication 5,
dans lequel ladite unité d'évaluation (14) est configurée pour régler de manière dynamique la première et/ou la seconde valeur absolue ou relative prédéterminée en fonction d'une condition physique du sujet.

9. Appareil de détection selon la revendication 8,
dans lequel ladite unité d'évaluation (14) est configurée pour régler de manière dynamique la première et/ou la seconde valeur absolue ou relative prédéterminée en fonction du fait que l'état est la réalisation d'une activité physique et/ou le type d'activité physique du sujet.

10. Appareil de détection selon la revendication 2,
dans lequel ladite unité de commande (16) est configurée pour commander audit au moins un capteur de démarrer une mesure avant un temps de mesure planifié.

11. Procédé de surveillance d'un signe vital d'un sujet, comprenant :
la mesure d'un signal de capteur représentant un signe vital ou étant lié à un signe vital,
l'évaluation dudit signal de capteur pour déterminer, sur la base de signaux de capteur préalablement mesurés, une mesure non concluante ou un signal de capteur non fiable, et
la commande à ladite mesure du signal de capteur de changer automatiquement l'intervalle de mesure sur la base du résultat de ladite évaluation,
**caractérisé en ce que** ladite commande est configurée pour commander à ladite mesure
- de répéter la mesure dudit signal de capteur pendant une période prédéterminée, représentant une durée de temps prédéterminée ou un nombre de fois prédéterminé, ou jusqu'à ce que la mesure soit concluante ou que le signal de capteur soit fiable, si une mesure non concluante ou un signal de capteur non fiable a été identifié(e),
- d'émettre l'un des signaux de capteur mesurés, même si toutes les mesures ont été non concluantes ou si tous les signaux de capteur mesurés sont non fiables ; et dans lequel, si toutes les mesures ont été non concluantes ou si tous les signaux de capteur mesurés sont non fiables, la commande est configurée en outre pour commander à ladite mesure
- de répéter la mesure du signal de capteur pendant une période prédéterminée supplémentaire, et
- de remplacer le signal de capteur émis par un nouveau signal de capteur mesuré au cours de ladite période prédéterminée supplémentaire, si au cours de ladite période prédéterminée supplémentaire, la mesure a été concluante ou un signal de capteur fiable a été mesuré.

12. Appareil de surveillance de patient pour surveiller des signes vitaux d'un sujet, comprenant
une interface de surveillance (42) configurée pour recevoir des signaux de capteur représentant un signe vital ou étant liés à un signe vital mesuré par au moins un capteur et pour transmettre des informations de commande audit au moins un capteur,
une unité d'évaluation (44) configurée pour évaluer des signaux de capteur reçus pour déterminer, sur la base des signaux de capteur reçus préalablement, des changements de l'état de santé du sujet et pour identifier une mesure non concluante ou un signal de capteur non fiable, et
une unité de commande (46) configurée pour générer des informations de commande pour la commande audit au moins un capteur de changer automatiquement l'intervalle de mesure sur la base du résultat de ladite évaluation,
**caractérisé en ce que**
ladite unité de commande (46) est configurée pour générer des informations de commande pour commander audit au moins un capteur
- de répéter la mesure du signal de capteur pendant une période prédéterminée, représentant une durée de temps prédéterminée ou un nombre de fois prédéterminé, ou jusqu'à ce que la mesure soit concluante ou que le signal de capteur soit fiable, si une mesure non concluante ou un signal de capteur non fiable a été identifié(e),
- d'émettre l'un des signaux de capteur mesurés, même si toutes les mesures ont été non concluantes ou si tous les signaux de capteur mesurés sont non fiables ; et dans lequel, si toutes les mesures ont été non concluantes ou si tous les signaux de capteur mesurés sont non fiables, l'unité de commande (16) est configurée en outre pour commander audit au moins un capteur
- de répéter la mesure du signal de capteur pendant une période prédéterminée supplémentaire, et
- de remplacer le signal de capteur émis par un nouveau signal de capteur au cours de ladite période prédéterminée supplémentaire, si au cours de ladite période prédéterminée supplémentaire la mesure a été concluante ou un signal de capteur fiable a été mesuré.

13. Procédé de surveillance de signes vitaux d'un sujet, comprenant
la réception de signaux de capteur représentant un signe vital ou étant liés à un signe vital mesuré par au moins un capteur,
l'évaluation des signaux de capteur reçus pour déterminer, sur la base de signaux de capteur préalablement reçus, une mesure non concluante ou un signal de capteur non fiable,
la génération d'informations de commande pour commander audit au moins un capteur de changer automatiquement l'intervalle de mesure sur la base du résultat de ladite évaluation, et
la transmission desdites informations de commande audit au moins un capteur,
dans lequel des informations de commande sont générées pour commander audit au moins un capteur
- de répéter la mesure dudit signal de capteur pendant une période prédéterminée, représentant une durée de temps prédéterminée ou un nombre de fois prédéterminé, ou jusqu'à ce que la mesure soit concluante ou que le signal de capteur soit fiable, si une mesure une mesure non concluante ou un signal de capteur non fiable a été identifié(e),
- d'émettre l'un des signaux de capteur mesurés, même si toutes les mesures ont été non concluantes ou si tous les signaux de capteur mesurés sont non fiables,
- de répéter la mesure du signal de capteur pendant une période prédéterminée supplémentaire, et
- de remplacer le signal de capteur émis par un nouveau signal de capteur mesuré au cours de ladite période prédéterminée supplémentaire, si au cours de ladite période prédéterminée supplémentaire la mesure a été concluante ou si un signal de capteur fiable a été mesuré.

14. Système de surveillance de patient pour surveiller des signes vitaux d'un sujet, comprenant
un appareil de surveillance de patient (40) selon la revendication 12, et
au moins un capteur (10, 50, 60) configuré pour mesurer un signal de capteur représentant un signe vital ou étant lié à un signe vital, ledit au moins un capteur comprenant une interface de capteur pour transmettre des signaux de capteur mesurés audit appareil de surveillance de patient et pour recevoir des informations de commande dudit appareil de surveillance de patient.

15. Support non transitoire lisible par un ordinateur ayant des instructions stockées sur celui-ci qui, lorsqu'elles sont réalisées sur un ordinateur, amènent l'ordinateur à exécuter les étapes du procédé selon la revendication 13.
